# EUROPEAN PATENT APPLICATION

(11) **EP 3 542 805 A1**
(43) Date of publication of application: **25.09.2019**
(21) Application number: 19169651.7
(22) Date of filing: 10.07.2014
(51) Int. Cl.: A61K 35/20, A61P 1/00, A61K 38/17, A61K 9/00, A23L 33/19, A23K 20/147, A23K 50/40

(54) **BETA-CASEIN A2 AND REDUCING OR PREVENTING SYMPTOMS OF LACTOSE INTOLERANCE**

(30) Priority: 12.07.2013 US 201361845480 P
(62) Divisional of application: 14823628.4
(71) Applicant: The A2 Milk Company Limited, Auckland 1010 (NZ)
(72) Inventor: CLARKE, Andrew John, 1052 Auckland (NZ); TRIVEDI, Malav Suchin, Florida, 33020 (US)
(74) Representative: EIP

(57) **Abstract**

Described herein is a composition for use in preventing or minimising epigenetic changes to one or more genes responsible for regulating the activity and levels of enzymes involved in digestive processes and gut function in an animal. The composition contains beta-casein. The beta-casein comprises at least 75% by weight of beta-casein variants having proline at position 67 of the beta-casein amino acid sequence.

## Description

This application is divided from European patent application number 14823628.4.

### TECHNICAL FIELD

The invention relates to the use of the milk protein beta-casein A2 for reducing or preventing the symptoms of lactose intolerance. In particular, the invention relates to milk and milk derived food products. The applicant has found that the consumption of milk and milk products that contain high levels of the protein beta-casein A2 and the avoidance of milk and milk products containing beta-casein A1 is beneficial for reducing or preventing the symptoms of lactose intolerance. Notably, the beneficial effect is immediate (acute) and additionally induces an ongoing (post-exposure to beta-casein A1) predisposition to preventing or reducing the symptoms of lactose intolerance on future exposure to lactose.

### BACKGROUND OF THE INVENTION

Lactose intolerance refers generally to a compromised ability to digest lactose. Lactose is a disaccharide carbohydrate comprising galactose and glucose monosaccharides. Lactose is found in milk and milk-derived dairy products. Human milk comprises about 9% lactose, whereas unprocessed bovine milk comprises about 4.7% lactose. Milk from goats, buffalo and sheep also contains lactose in the range 4.5-5.0%. The digestion of lactose is the hydrolysis (or splitting) of lactose into galactose and glucose by a lactase enzyme.

Individuals who are lactose intolerant lack sufficient levels of lactase in their digestive system. Lactose cannot be absorbed through the wall of the small intestine into the bloodstream and therefore, if not broken down by a lactase, passes intact into the colon. Bacterial fermentation of lactose in the colon produces a large amount of gas. Further, unabsorbed carbohydrates and fermentation products raise the osmotic pressure of the colon causing an increased flow of water into the bowel. Lactose intolerance therefore can cause a range of symptoms including abdominal bloating and cramps, flatulence, diarrhoea, nausea, rumbling stomach, or even vomiting. These symptoms normally occur about 30 minutes to 2 hours following consumption of lactose.

Early infant mammals produce lactase, but this production normally ceases after weaning. However, some human populations have developed lactase persistence where lactase production continues into adulthood. The degree of lactase persistence in different populations is thought to be a result of natural selection favouring those cultures in which dairy products are available as a food source.

Lactose intolerance is not an absolute in that the amount of lactose that can be tolerated varies from person to person. In general, a lactose intolerant individual must, by trial and error, work out how much lactose they can tolerate. This is usually done by controlling levels of dietary lactose or by avoiding dietary lactose altogether. In some cases, enzymatic lactase supplements may be used. Plant-based milks or milk derivatives can be used because they are inherently free of lactose, e.g. soy milk, rice milk, almond milk, coconut milk, oat milk, hemp milk and peanut milk. There are also many lactose-free or reduced-lactose foods available. Despite the availability of such foods, the avoidance of milk or dairy products in diet is often difficult.

The link between the consumption of milk (and other dairy products) and the *symptoms* of lactose intolerance is well-known. However, in the absence of a medical diagnosis specifically for lactose intolerance, many individuals mistakenly consider themselves to be lactose intolerant because they connect the symptoms they suffer with the consumption of milk or other dairy products. The symptoms may, in fact, be due to other milk components exacerbating otherwise negligible or unnoticeable effects. Proteins are an example of a component that may cause or exacerbate such symptoms.

Milk, mainly bovine milk, consumed in populations throughout the world, is a major source of protein in human diets. Bovine milk typically comprises around 30 grams per litre of protein. Caseins make up the largest component (80%) of that protein, and beta-caseins make up about 37% of the caseins. In the past two decades the body of evidence implicating casein proteins, especially beta-caseins, in a number of health disorders has been growing.

The beta-caseins can be categorised as beta-casein A1 and beta-casein A2. These two proteins are the predominant beta-caseins in the milk consumed in most human populations. Beta-casein A1 differs from beta-casein A2 by a single amino acid. A histidine amino acid is located at position 67 of the 209 amino acid sequence of beta-casein A1, whereas a proline is located at the same position of beta-casein A2. This single amino acid difference is, however, critically important to the enzymatic digestion of beta-caseins in the gut. The presence of histidine at position 67 allows a protein fragment comprising seven amino acids, known as beta-casomorphin-7 (BCM-7), to be produced on enzymatic digestion. Thus, BCM-7 is a digestion product of beta-casein A1. In the case of beta-casein A2, position 67 is occupied by a proline which hinders cleavage of the amino acid bond at that location. Thus, BCM-7 is not a digestion product of beta-casein A2.

Other beta-casein variants, such as beta-casein B and beta-casein C, also have histidine at position 67, and other variants, such as A3, D and E, have proline at position 67. But these variants are found only in very low levels, or not found at all, in milk from cows of European origin. Thus, in the context of this invention, the term beta-casein A1 refers to any beta-casein having histidine at position 67, and the term beta-casein A2 refers to any beta-casein having proline at position 67.

BCM-7 is an opioid peptide and can potently activate opioid receptors throughout the body. BCM-7 has the ability to cross the gastrointestinal wall and enter circulation enabling it to influence systemic and cellular activities via opioid receptors. The applicant and others have previously determined a link between the consumption of beta-casein A1 in milk and milk products and the incidence of certain health conditions including type I diabetes (WO 1996/014577), coronary heart disease (WO 1996/036239) and neurological disorders (WO 2002/019832).

There has been speculation that BCM-7 can also affect digestive function. It has been reported that opioid receptors play a role in controlling gastrointestinal function, including regulating gastrointestinal motility, mucus production and hormone production. (for example, Mihatsch, W.A, et al., Biol. Neonate, 2005, 87(3):160-3). The caseins found in milk are thought to be associated with inhibiting intestinal motility, which can lead to constipation (Gunn T.R. and Stunzer D., NZ Med. J., 1986, 99(813):843-6) and research on casomorphins and synthetic casomorphin derivatives indicates that BCM-7 contributes to this opioid receptor mediated effect (Charlin V. et al., Rev. Med. Chil., 1992, 120(6):666-9). However, while there is some *in vitro* evidence for a link between casomorphins and transit time in the intestines, it is apparent that the effect cannot necessarily be extrapolated to an *in vivo* effect in humans. For example, at least one study failed to demonstrate a relationship between beta-casein A1 or beta-casein A2 consumption and constipation (Crowley, E.T., Nutrients, 2013, 5, 253-266). Additionally, BCM-7 has been shown to stimulate the production of mucus via mu-opiate receptor mediated pathways (Zoghbi, S., Am. J. Physiol. Gastrointest. Liver Physiol., 2006, 290(6):G1105-13) and to modulate the proliferation of lamina propia lymphocytes (Elitsur, Y. and Luk, G.D., Clin. Exp. Immunol., 1991, 85(3):493-7) which are cells associated with the immune system. More recently beta-casein A1 has been reported to cause inflammation of tissue in the gastrointestinal tract (UI Haq, M.R., et al., Eur. J. Nutr., 2013; Barnett, M.P.G., et al., Int. J. Food Sci. Nutr., 2014). Inflammation induced by beta-casein A1 derived BCM-7 was demonstrated to have downstream effects on epigenetic DNA modification and subsequent gene expression of the affected tissue (Trivedi, M.S., et al., J. Nut. Bio., 2014).

The above reports indicate links between caseins and casomorphins (including BCM-7) and gastrointestinal function. These reports are based on studies using milk proteins or caseins generally or on studies using BCM-7 itself. However, to date, there has been no report directly linking the consumption of beta-casein A1 to gastrointestinal function and the symptoms of lactose intolerance in particular. In addition, there have been anecdotal reports (online and media) from consumers having unknown or unconfirmed conditions referring to improvements in gastrointestinal function after drinking milk high in beta-casein A2 (and conversely low in beta-casein A1), but these are non-scientific reports and they are non-specific as to the cause of any improvement in function. Furthermore, there are also many anecdotal reports of no improvement effect on consumption of such milk. These reports are conflicting in that they include reports across the digestion effect continua of motility and stool consistency, from constipation through to diarrhoea. Conclusions cannot be made with confidence from anecdotal reports, particularly in the case of food products and physiological function where the number of variables that can potentially impact on outcomes is very large.

The applicant has now found conclusive scientific evidence for a direct link between the consumption of beta-casein A1 and the symptoms of lactose intolerance. Given the myriad of factors in human diet that can influence bowel health, and that milk and milk products contain a wide array of protein components and other components, the applicant's finding of a clear direct association between beta-casein A1 consumption and the symptoms of lactose intolerance is surprising. Importantly, the applicant has found evidence, not only of an acute and undesirable response to the consumption of beta-casein A1, but also of an ongoing (post-exposure to beta-casein A1 or BCM-7) response in that the consumption of beta-casein A1, and resultant production of BCM-7, can induce genetic changes in an animal that lead to lower levels of lactase and consequently an increased likelihood of causing symptoms of lactose intolerance on future exposure to lactose.

It is therefore an object of the invention to provide a method for reducing or preventing the symptoms of lactose intolerance, or to at least provide a useful alternative to existing methods.

### SUMMARY OF THE INVENTION

In a first aspect of the invention there is provided a composition for use in preventing or minimising epigenetic changes to one or more genes responsible for regulating the activity and levels of enzymes involved in digestive processes and gut function in an animal, wherein the composition contains beta-casein and wherein the beta-casein comprises at least 75% by weight of beta-casein variants having proline at position 67 of the beta-casein amino acid sequence.

In a second aspect of the invention there is provided the use of a composition for preventing or reducing the symptoms of lactose intolerance in an animal, where the composition contains beta-casein, and where the beta-casein comprises at least 75% by weight beta-casein A2.

In a third aspect of the invention there is provided a composition for preventing or reducing the symptoms of lactose intolerance in an animal which composition contains beta-casein and where the beta-casein comprises at least 75% by weight beta-casein A2.

In another aspect of the invention there is provided the use of milk in the manufacture of a composition for preventing or reducing the symptoms of lactose intolerance an animal where the milk contains beta-casein and where the beta-casein comprises at least 75% by weight beta-casein A2.

In another aspect there is provided the use of beta-casein A2 in the manufacture of a composition for preventing or reducing the symptoms of lactose intolerance in an animal where the composition comprises at least 75% by weight beta-casein A2. The beta-casein A2 is preferably a component of milk. The milk is preferably bovine milk.

In a further aspect of the invention there is provided a method of preventing or reducing the symptoms of lactose intolerance in an animal comprising the consumption by the animal of a composition containing beta-casein, or providing the composition to the animal for consumption, where the beta-casein comprises at least 75% by weight beta-casein A2.

The amount of beta-casein A2 may be any amount in the range of 75% to 100% by weight of the beta-casein, for example at least 90% or even 100%.

In certain embodiments of the invention, the composition is milk or a milk product. The milk may be milk powder or liquid milk. The milk product may be cream, yoghurt, quark, cheese, butter, ice cream, or any other milk product.

The symptoms of lactose intolerance may be, although are not limited to, abdominal bloating and cramps, flatulence, diarrhoea, nausea, rumbling stomach, and vomiting.

The response to consumption of the composition by the animal may be an acute response and may additionally induce a predisposition in the animal to preventing or reducing the symptoms of lactose intolerance on future exposure to lactose.

In most embodiments of the invention, the animal is a human. However, in other embodiments, the animal may be a dog, cat, or any other domestic animal where feed is supplemented with milk.

### BRIEF DESCRIPTION OF THE FIGURES

Figure 1 shows gastrointestinal transit times in rats fed the diets of Example 1.
Figure 2 shows duodenum lactase activity in rats fed the diets of Example 1.
Figure 3 shows colon myeloperoxidase activity in rats fed the diets of Example 1.
Figure 4 shows morphine and BCM-7 concentration dependent uptake of cysteine in neuronal cells and GI epithelial cells.
Figure 5 shows time-dependent uptake of cysteine in neuronal cells and GI epithelial cells.
Figure 6 shows the involvement of µ-opioid receptor in mediating the effects of BCM-7 and morphine on cysteine uptake.
Figure 7 shows the effects of BCM-7 and morphine on cysteine levels, GSH/GSSG and SAM/SAH over time.
Figures 8 and 9 show the influence of BCM-7 on CpG methylation in the genes implicated in lactose metabolism and lactose synthesis.
Figure 10 shows the levels of LCT gene, which codes for lactase, in the small intestine of NOD mice fed a beta-casein A1 or beta-casein A2 diet for 10 and 20 weeks.

### DETAILED DESCRIPTION

The invention relates to a composition containing the protein beta-casein and its use for reducing or preventing the symptoms of lactose intolerance. Importantly, the beta-casein is the A2 variant of beta-casein, or makes up at least 75% by weight of the total beta-casein variants present in the composition. The importance of the predominance of the A2 variant in the composition is due to the fact that the applicant has shown that there is a direct link between the A1 variant and the symptoms of lactose intolerance in humans. The applicant has also shown that the presence of milk protein containing high levels of the A2 variant in the duodenum beneficially stimulates lactase activity. Therefore, an improvement in bowel health can be expected if the consumption of the A1 variant is avoided and the A2 variant is consumed instead.

The term "symptoms of lactose intolerance" as used in this specification is intended to mean any one or more of a range of symptoms that includes abdominal bloating and cramps, flatulence, diarrhoea, nausea, rumbling stomach, and vomiting, which symptoms may be acute, transitional or chronic.

The term "acute" as used in this specification, unless otherwise indicated, is intended to mean during the period of time from consumption of beta-casein A1 to exit of beta-casein A1 or BCM-7 from the gut (typically 8-20 hours after consumption).

Since the primary, if not only, source of beta-caseins in the diet of most human populations is milk or products derived from milk, and since most milk consumed contains a mixture of the A1 and A2 variants of beta-casein only, the consumption of milk (or products made from such milk) having a high content of the A2 variant will necessarily mean that the consumption of the A1 variant is low. Following from this, if the only dietary source of beta-casein contains the A2 variant and no other variant, the dietary intake of the A1 variant is eliminated and the adverse the symptoms of lactose intolerance arising from beta-casein A1 consumption can therefore also be expected to be eliminated.

Accordingly, the invention of this application is based on the reduction or elimination of beta-casein A1 in the diet, and the promotion of beta-casein A2, and this is achieved by ensuring that the beta-casein in beta-casein containing food compositons, especially milk and milk products, is predominantly or even exclusively beta-casein A2.

Ideally, the beta-casein in the composition is 100% beta-casein A2. The complete elimination of beta-casein A1 therefore maximises the associated health benefit by reducing or eliminating altogether the the symptoms of lactose intolerance. However, the symptoms may be reduced in any composition where the beta-casein is predominantly beta-casein A2, for example, any amount between 75% by weight and 100%, including but not limited to 80%, 90%, 95%, 98% and 99% by weight.

The composition of the invention is typically milk, but may also be any milk-derived product such as cream, yoghurt, quark, cheese, butter, or ice cream. The composition may also be a non-milk product containing beta-casein that has been obtained from milk. The composition may be beta-casein itself, or may be prepared from beta-casein, which beta-casein may be in solid form such as powder or granules or in the form of a solid cake.

While the milk may be obtained from any mammal, including humans, goats, pigs and buffalo, in preferred embodiments of the invention the milk is bovine milk.

The milk may be in the form of fresh milk, milk powder, liquid milk reconstituted from a powder, skim milk, homogenised milk, condensed milk, evaporated milk, pasteurised milk or non-pasteurised milk, or any other form of milk.

The composition of the invention is applicable for consumption by humans primarily, but it should be appreciated that the health benefit is also relevant for some other animals such as cats, dogs and other domestic animals.

Support for the invention is found in the experiments described in the Examples.

Example 1 sets out the feeding methodology for the rat studies of Examples 2 to 4. The diets are shown in Table 1. The A1 milk diet is based on a formulation where all the beta-casein in the diet is beta-casein A1. The A2 milk diet is based on a formulation where all the beta-casein in the diet is beta-casein A2. The control diet is based on a formulation where the protein content is egg white.

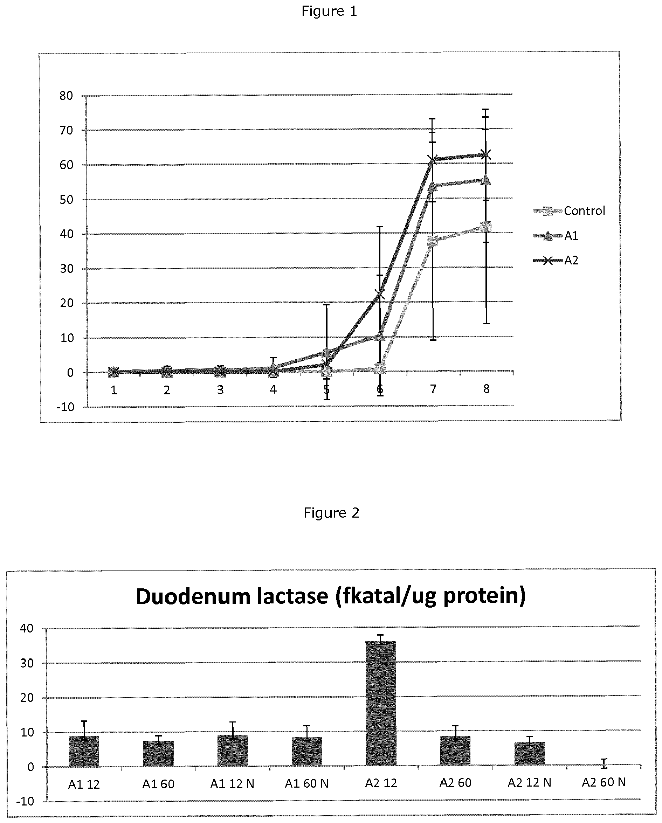

Example 2 describes an investigation into gastrointestinal transit time (GITT) in rats fed the different diets of Example 1. Titanium dioxide (TiO₂), used as a tracer, was administered orally to animals following 12 hours of feeding. Recovery of the TiO₂ is shown in Figure 1 as % recovery versus time (hours). Rats fed the A1 diet showed delayed transit relative to rats fed the A2 diet, with both groups showing delay relative to rats fed the control diet. This is consistent with beta-casein A1 having higher general opioid activity than beta-casein A2 due to the release of BCM-7. The symptoms of lactose intolerance are linked to the bacterial fermentation of lactose in the gut. The bacterial count during fermentation increases exponentially with GITT. Thus, if motility is decreased by a factor of two, there will be a four-fold increase in the rate of fermentation and therefore the manifestation of lactose intolerance symptoms. Example 2 is therefore evidence that a diet containing beta-casein A1, relative to a diet containing beta-casein A2, is more likely to contribute to a delay in GITT and give rise to symptoms of lactose intolerance.

Example 3 shows that lactase activity in the duodenum following acute feeding (after 12 hours) is strongly elevated relative to chronic feeding (after 60 hours) for rats fed the 100% A2 diet, but not the 100% A1 diet. This means that beta-casein A2, and milk or milk products containing beta-casein A2, may be used to promote healthy gastrointestinal function and the digestion of lactose in diet or to relieve or eliminate the symptoms of lactose intolerance experienced when milk and dairy products are consumed. It is thought that diets containing beta-casein A2 stimulate the secretion or activity of lactase, but diets containing beta-casein A1 do not. This is most likely due to a differential effect that the digestion products of beta-casein A1 and beta-casein A2 have on tissue inflammation and function following the stimulation of enzyme secretion by a bolus of milk protein entering the small intestine.

Example 4 relates to the effect of beta-casein A1 and beta-casein A2 diets on myeloperoxidase (MPO) activity in the colon of rats. MPO activity is a marker for inflammation (Krawisz, et al., Gastroenterology, 1984, 87(6):1344-1350 and Dommels, Y.E.M., et al., Genes Nutr., 2007, 2(2):209-223). It was found that colon MPO activity increased in beta-casein A1-fed rats compared to beta-casein A2-fed rats indicating an increased level of neutrophil cells in beta-casein A1-fed rats, which is in turn an indicator of inflammatory response. The effect was not observed in rats treated with naloxone (a known opioid receptor antagonist), demonstrating that the effect is mediated through the interaction of BCM-7 with mu-opiate receptors. Inflammation of the colon causes increased susceptibility or sensitivity to the symptoms of lactose intolerance.

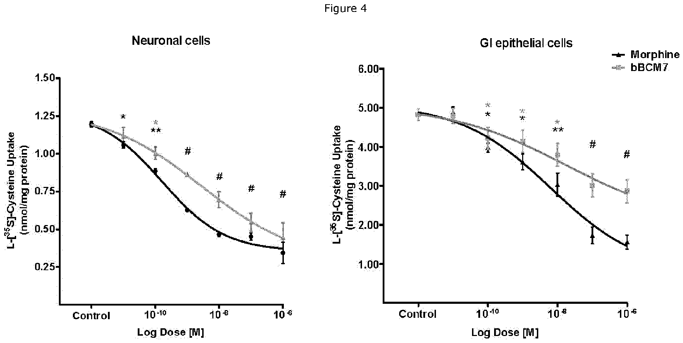

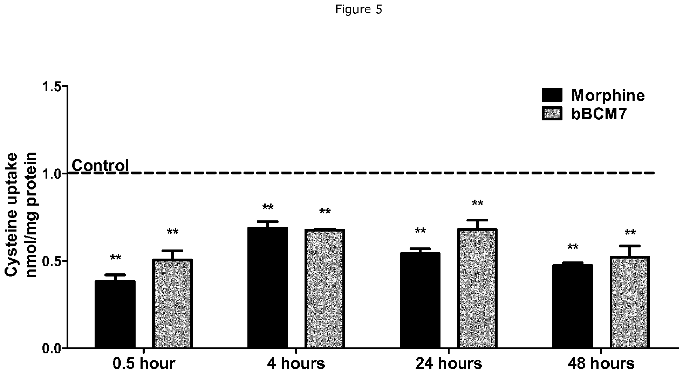

Example 5 indicates that BCM-7 can inhibit the uptake of cysteine in a concentration- dependent manner. Morphine showed greater efficacy than BCM-7 with IC50 values of 0.16 and 1.31 nM (respectively) in neuronal cells and 6.38 and 15.95 nM (respectively) in GI epithelial cells (Figure 4). Inhibition of cysteine uptake was fully developed after 30 minutes and was sustained through 48 hours of morphine or BCM-7 exposure (Figure 5). This indicates a long term chronic effect on cysteine uptake after single exposure to BCM-7. The blockade in the presence of a selective µ-antagonist and not a delta opioid receptor showed that these effects were µ-opioid receptor mediated.

Food-derived peptides are reported to alter redox metabolism including the levels of glutathione which can regulate the levels of S-adenosylmethionine (SAM) in cells. SAM is the universal methyl donor for mediating DNA methylation changes. These changes are part of epigenetic regulatory memory and can regulate the levels of gene expression to maintain homeostasis. Importantly, these changes can be highly stable and have the potential to interfere with gene expression/repression and hence permanently alter gene levels. The levels of glutathione can therefore impact on the pathways in which the genes play an important role, such as lactose synthesis and metabolism pathways. Hence, epigenetic changes induced by BCM-7 via the redox based signalling pathway can affect the regulatory genes responsible for lactose synthesis and metabolism and therefore affect lactose levels in the body. The body is attuned to absorb, metabolise, clear or store a certain total level of lactose. If the level is altered under the influence of BCM-7, the body's capacity to regulate lactose levels may be saturated and hence induce downstream pathophysiological subclinical or clinical effects.

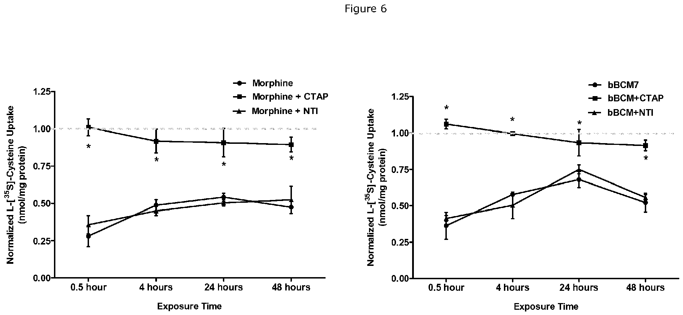

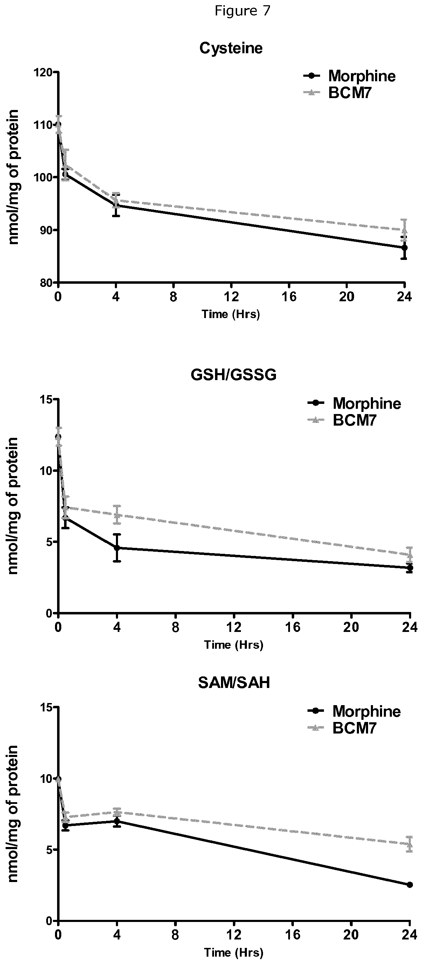

Example 6 shows that BCM-7 and morphine cause time-dependent decreases in both cysteine and glutathione (GSH) levels. The intracellular levels of cysteine in neuronal cells and the redox status of the cells (reflected by the ratio of GSH to its oxidised form glutathione disulphide (GSSG)), were also decreased (Figure 6), indicating the possibility of an oxidative stress condition. Further, methylation capacity (indicated by the SAM/SAH ratio) was also affected by BCM-7 treatment at different time points (Figure 7). Hence, BCM-7 induces a reduction in major intracellular antioxidant levels, specifically GSH levels. Reduced GSH levels are known to induce chromatin modifications via the oxidative-stress signalling pathway by regulating the SAM levels.

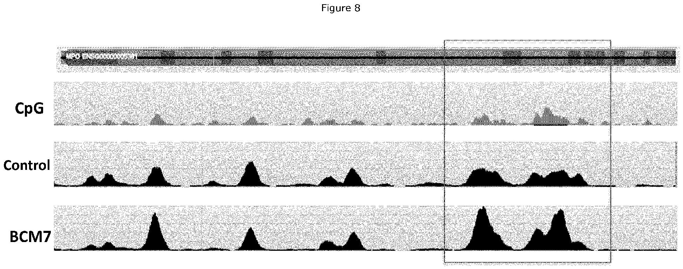

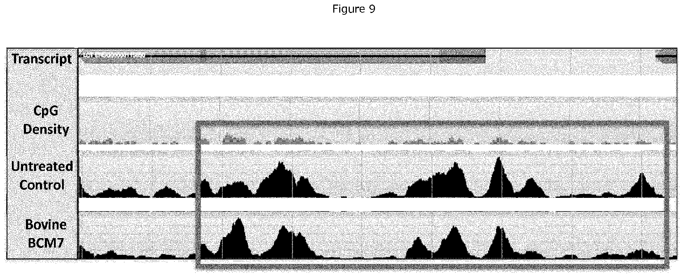

Example 7 investigates the DNA methylation levels induced by BCM-7. Figure 8 shows DNA methylation changes in MPO, one of the genes responsible for mediating the inflammatory response influenced by BCM-7. Changes in redox status are shown to cause long term changes in the epigenetic status of the inflammatory genes. This is equivalent to a memory of the molecular insults, potentially contributing to long-term chronic changes and inflammatory responses to lactose intolerance. Thus, BCM-7 not only alters MPO activity, as evident from the beta-casein A1 feeding studies, but also alters the epigenetic status of the MPO gene and therefore has an ongoing and long term effect on lactose synthesis and metabolism. The downstream effects of altered lactose levels can include gastric dysfunction and digestive problems. As indicated in Figure 9, BCM-7 alters the epigenetic state of enzymes such as lactase which is involved in lactose metabolism and degradation. This may lead to accumulated lactose levels, and regulated concentrations of lactose which may or may not be tolerable depending on the individual. If not tolerable, changes in the digestive functions and gut inflammation can be expected.

BCM-7 also affects the enzymes involved in digestive function as shown in Table 4. B4GALT2, LGALS12 and B4GALT1 are genes that code for enzymes involved in galactose metabolism. Galactose is an important intermediate in the synthesis of lactose. Similarly, GKN1, GALK2, GALR2, GALT, and GALR1 code for enzymes that are involved in regulating the levels of galactose and hence indirectly regulate the levels of lactose. Changes in the activity of these enzymes can indirectly lead to changes in the levels of lactose. BCM-7 alters the enzymatic activity of these enzymes by regulating the epigenetic status of these genes. This is mediated by the mechanistic regulation of the redox status. These changes ultimately skew lactose levels, not only in the acute stage, but could also have a long term effect because of epigenetic changes that may even be passed to the next generation.

BCM-7 not only mediates changes in lactose synthesis and metabolism, but can also regulate the activity and levels of enzymes involved in digestive processes and gut function. Genes for enzymes such as cholecystokinin, motilin, secretase, and oxytocin are shown to have an altered epigenetic status influenced by BCM-7. This would directly impact on the gastrointestinal function and digestive capabilities of an individual. Thus, BCM-7 would contribute to the symptoms of altered gut motility, digestive abnormalities, flatulence and diarrhoea, all of which are symptoms of lactose intolerance.

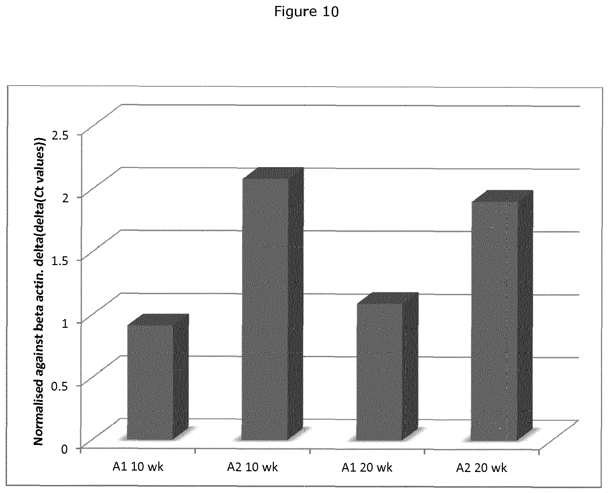

The downstream effects of epigenetic changes on lactase enzyme levels were further confirmed on investigation into mRNA levels in qPCR (Example 8). NOD mice were fed beta-casein-A1 or beta-casein A2 enriched diets post-weaning. Following euthanasia intestinal samples were dissected and collected. RNA was isolated from these samples and PCR performed using primers specific for lactase enzyme levels in the small intestine. As indicated in Figure 10, the mRNA levels of lactase enzymes were higher in the small intestine of mice fed the beta-casein A2 diet for 10 and 20 weeks compared with the levels of lactase mRNA in the small intestine of mice fed the beta-casein A1 diet over the same time periods. Having higher levels of lactase enzyme, beta-casein A2 fed mice would clear the lactose from the digestive system and would only allow a certain level of lactose to be available. Symptoms associated with lactose intolerance are therefore avoided. In contrast, having lower levels of lactase mRNA in small intestine, mice fed the beta-casein A1 diet may allow higher levels of lactose to build and therefore cause symptoms of lactose intolerance.

These studies represent the first clear scientific evidence of a link between beta-casein A1 consumption and the symptoms of lactose intolerance, and additionally that beta-casein A2 consumption (relative to beta-casein A1 consumption) induces a beneficial predisposition to the prevention or reduction of the symptoms of lactose intolerance on future exposure to lactose. Previously, inconclusive and conflicting anecdotal reports and studies relating to BCM-7 (rather than beta-casein A1 itself) had lead to confusion among those skilled in the art, with many believing there was no such link. Through the applicant's finding, an alternative potential solution is provided to the problems that have been suffered by many people who considered themselves to be lactose intolerant, i.e. the avoidance of beta-casein A1 in diet. This can be achieved by obtaining milk having a beta-casein content that is predominantly beta-casein A2 and producing products derived from that milk, and making that milk and those products available for the purpose of reducing or preventing the symptoms of lactose intolerance.

The milk of cows can be tested for the relative proportions of beta-casein A1 and beta-casein A2. Alternatively, cows can be genetically tested for their ability to produce milk containing beta-casein A1 or beta-casein A2 or a combination of both. These techniques are well-known.

The invention has distinct advantages over existing methods for avoiding the symptoms of lactose intolerance. Most existing methods rely on dietary modifications, many of which often have limited or no real success. The present invention provides a solution that is comparatively easy to manage, i.e. avoidance of milk or milk products that contain beta-casein A1 and ensuring that milk and milk products in the diet contain beta-casein that is predominantly beta-casein A2, preferably 100% beta-casein A2. The invention avoids any need for wholesale dietary mofications such as the avoidance of dairy products or other common food products.

Any reference to prior art documents in this specification is not to be considered an admission that such prior art is widely known or forms part of the common general knowledge in the field.

As used in this specification, the words "comprises", "comprising", and similar words, are not to be interpreted in an exclusive or exhaustive sense. In other words, they are intended to mean "including, but not limited to".

The invention is further described with reference to the following examples. It will be appreciated that the invention as claimed is not intended to be limited in any way by these examples.

### EXAMPLES

### Example 1: Feeding Methodology

Seventy two weaned (four week old) male Wistar rats were used. Following a 7-day acclimatisation period on a control diet, the rats were fed for either 12 or 60 hours with one of three diets: 100% A1 diet, 100% A2 diet, control diet (n=6 per treatment). The protein component of the diets were derived from skim milk (for the A1 and A2 diets) and on egg white (for the non-milk protein control diet), and were balanced for energy and macronutrient composition (see Table 1). Fifteen minutes before the end of the time period, rats received either naloxone or saline (control) via intra-peritoneal injection, and were then orally gavaged with a non-digestible tracer, titanium dioxide. Faecal and urine samples were collected at 7 time points over the following 24 hours, and stored at -20 °C (faecal) or -80 °C (urine) until they were analysed.

**Table 1: Composition of diets**

| **Product** | **A1 milk diet** | | **A2 milk diet** | | **Control diet** | |
|---|---|---|---|---|---|---|
| Ingredient | gm | kcal | gm | kcal | gm | kcal |
| Casein | 0 | 0 | 0 | 0 | 0 | 0 |
| A1 milk powder | 475 | 1691 | 0 | 0 | 0 | 0 |
| A2 milk powder | 0 | 0 | 468 | 1687 | 0 | 0 |
| DL-methionine | 3 | 12 | 3 | 12 | 0 | 0 |
| Egg whites (dried) | 0 | 0 | 0 | 0 | 200 | 800 |
| Corn starch | 150 | 600 | 150 | 600 | 153 | 612 |
| Sucrose | 288 | 1152 | 294 | 1176 | 500 | 2000 |
| Cellulose, BW200 | 50 | 0 | 50 | 0 | 50 | 0 |
| Corn oil | 45.2 | 406.8 | 43 | 387 | 50 | 450 |
| Mineral mix S10001 | 35 | 0 | 35 | 0 | 35 | 0 |
| Biotin, 1% | 0 | 0 | 0 | 0 | 0.4 | 0 |
| Vitamin mix V10001 | 10 | 40 | 10 | 40 | 10 | 40 |
| Choline bitartrate | 2 | 0 | 2 | 0 | 2 | 0 |
| Total | 1058.2 | 3902 | 1055 | 3902 | 1000.4 | 3902 |

### Example 2: Gastrointestinal Transit Time

Gastrointestinal transit time (GITT) was measured in rats fed according to Example 1. Titanium dioxide (TiO₂) was used as a tracer administered orally to animals following 12 hour of feeding the 100% A1 diet, the 100% A2 diet, or the control diet. The results are shown in Table 2 and in Figure 1. Recovery data is represented as the % TiO₂ recovery versus time (hours). Rats fed the A1 diet showed delayed transit relative to rats fed the A2 diet, with both groups showing delay relative to rats fed the control diet.

**Table 2: GI Transit Times**

| **Time** | **Control** | **SD** | **A1** | **SD** | **A2** | **SD** |
|---|---|---|---|---|---|---|
| 1 | 0.001 | 0.002 | 0.171 | 0.406 | 0.001 | 0.002 |
| 2 | 0.006 | 0.011 | 0.514 | 1.218 | 0.011 | 0.024 |
| 3 | 0.028 | 0.047 | 0.522 | 1.221 | 0.033 | 0.043 |
| 4 | 0.029 | 0.046 | 1.189 | 2.854 | 0.056 | 0.036 |
| 5 | 0.064 | 0.071 | 5.624 | 13.713 | 2.048 | 4.162 |
| 6 | 0.758 | 1.196 | 10.343 | 17.419 | 22.188 | 19.698 |
| 7 | 37.605 | 28.549 | 53.530 | 15.513 | 61.024 | 11.983 |
| 8 | 41.716 | 28.082 | 55.296 | 18.084 | 62.482 | 13.170 |

### Example 3: Lactase Activity

Frozen powdered duodenum tissue samples were homogenised in ice-cold deionised water (1:5 wt/vol), then centrifuged at 2,200g for 30 minutes at 4. The supernatant was harvested and further diluted (1:25) with deionised water. The samples were incubated with lactose and the liberated glucose determined using a glucose-oxidase kit (Sigma) and measured with a microplate reader. Table 3 and Figure 2 show the results for duodenal lactase for both acute (12 hour) and chronic (60 hour) fed groups of rats. Duodenal lactase activity was elevated in acute fed A2 groups, relative to chronic fed A2 groups and to both acute and chronic fed A1 groups.

**Table 3: Lactase activity for acute and chronic fed groups**

| | **Duodenum lactase (fkatal/ ug protein)** | **Std Dev** |
|---|---|---|
| **A1 12** | 8.94 | 3.87 |
| **A1 60** | 7.35 | 2.19 |
| **A1 12 N** | 8.99 | 3.86 |
| **A1 60 N** | 8.42 | 2.59 |
| **A2 12** | 35.97 | 32.23 |
| **A2 60** | 8.45 | 1.92 |
| **A2 12 N** | 6.55 | 2.76 |
| **A2 60 N** | no data | no data |

### Example 4: MPO Activity

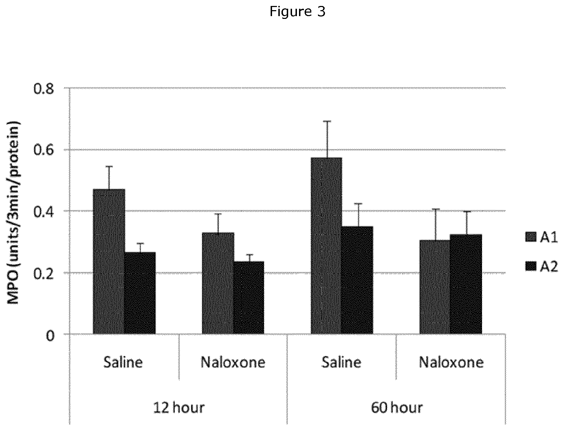

Colon tissue from the rats fed according to Example 1 was quantified for myeloperoxidase (MPO) activity based on an established method (Grisham, M.B., et al., Methods Enzymol., 1990, 186:729-742). Colon tissue (50 mg) was homogenised, partitioned via centrifugation, ruptured by ultrasonic probe and subjected to a freeze-thaw cycle. Endogenous MPO catalyses H₂O₂-dependent oxidation of 3,3',5,5'-tetramethyl-benzidine substrate measured colourimetrically at 562 nm. Activity was normalised by a bicinchoninic acid (BCA) (Smith, P.K., et al., Anal. Biochem., 1985, 150(1):76-85) protein determination for the same homogenate. The results are shown in in Figure 3. Relative to A1 fed animals A2 animals demonstrated a significantly lower level of MPO activity following acute feeding. This was persistent and further increased with chronic feeding and completely reversible by the oral administration of naloxone.

### Example 5: Effect of BCM-7 on Uptake of Cysteine

Radiolabelled [³⁵S]-cysteine uptake assay was performed in Caco-2-GI epithelial cells and neuronal cells, in the presence of BCM-7 released from beta-casein A1, and compared against untreated controls as well as against morphine (a prototypical opioid receptor agonist). Pre-treatment in cells was performed for different time points for 30 min, 4 h, 24 h and 48 h as described previously (Trivedi M., et al.; Mol. Pharm., 2014). SH-SY5Y human neuronal cells and Caco-2 Gut epithelial cells were plated in six-well plates and were pretreated with drugs and incubated for various times prior to measuring uptake. Media were aspirated and cells were washed with 600 µL of HBSS at 37 °C. Non-radioactive HBSS was aspirated, replaced with 600 µL of 37 °C HBSS containing [³⁵S]-cysteine (1 µCi/1 mL), 10 µM unlabelled cysteine and 100 µM DTT, and the cells were incubated for 5 min. The [³⁵S]-cysteine/HBSS mixture was aspirated and treatment was terminated by two washes with ice-cold HBSS. Cells were then lysed with 600 µL of dH₂O, scraped, collected in 1.5 mL microcentrifuge tubes, and sonicated for 10 s. 100 µL of each sample was aliquoted for protein assay. 200 µL of each sample (in triplicate) was aliquoted into scintillation vials with 4 mL of scintillation fluid, vortexed, and counted for radioactivity (normalised against protein content). Additionally, the cysteine uptake effects of morphine and BCM-7 were also characterised in the presence of D-Phe-Cys-Tyr-D-Trp-Arg-Thr-Pen-Thr (CTAP), a selective µ-antagonist, and the delta antagonist naltrindole (NTI). The results are shown in Figures 4, 5 and 6. The symbol * used in these figures indicates a statistically significant difference (p<0.05) compared against the untreated control, and the symbol # indicates a statistically significant difference (p< 0.005) compared against the untreated control.

### Example 6: Effect of BCM-7 on GSH and SAM Levels

This example investigated whether decreases in cysteine uptake as observed in Example 5 could translate into GSH changes and affect antioxidant levels. The intracellular levels of GSH were measured with BCM-7 as well as with morphine for different times (30 min, 4h, 24h) using HPLC and an electrochemical gradient detection method (Hodgson et al., J. Alzh. Dis. 2013, Trivedi M., et al., Mol. Pharm. 2014). SH-SY5Y neuronal cells were grown to confluence in α-MEM. Media was aspirated and the cells were washed twice with 1 mL of ice cold HBSS. HBSS was aspirated and 0.6 mL ice cold dH₂O was added to the cells. The cells were scraped from the flask/dish and suspended in dH₂O. The cell suspension was sonicated for 15 s on ice and 100 µL of the suspension was used to determine the protein content. The remaining lysate was added to a microcentrifuge tube and an equal volume of 0.4 N perchloric acid was added, followed by incubation on ice for 5 min. Samples were centrifuged at 5,000 × g and the supernatant transferred to new microcentrifuge tubes. 100 µL of each sample was added to a conical micro-autosampler vial and kept at 4 °C in the autosampler cooling tray. 10 µL of each of these samples was injected into the HPLC system.

The separation of redox and methylation pathway metabolites was accomplished using an Agilent Eclipse XDB-C8 analytical column (3 × 150 mm; 3.5 µm) and an Agilent Eclipse XDB-C8 (4.6 × 12.5 mm; 5 µm) guard column. Two mobile phases were used. Mobile Phase A: 0% acetonitrile, 25 mM sodium phosphate, 1.4 mM 1-octanesulfonic acid, adjusted to pH 2.65 with phosphoric acid. Mobile Phase B: 50% acetonitrile. The flow rate was initially set at 0.6 mL/min and a step gradient was used: 0-9 min 0% B, 9-19 min 50% B, 19-30 min 50% B. The column was then equilibrated with 5% B for 12 min prior to the next run. Temperature was maintained at 27 °C. The electrochemical detector used is an ESA CoulArray with BDD Analytical cell Model 5040 and the operating potential was set at 1500 mV. Sample concentrations were determined from the peak areas of metabolites using standard calibration curves and ESA-supplied HPLC software. Sample concentrations were normalised against protein content. In some cases samples were diluted in mobile phase as needed or up to 50 µl of sample was injected to assure that thiol levels were within the range of the standard curve. The results are shown in Figure 7.

### Example 7: Effect of BCM-7 on DNA Methylation levels

Global DNA methylation levels induced by BCM-7 were investigated using methyl-CpG binding domain (MBD) protein-enriched genome sequencing (MBD-seq) as described previously (Trivedi M., et al., Mol. Pharm. 2014), whereas mRNA translation microarray data was obtained using Agilent V3 microarray chip, from non-treated control SH-SY5Y cells and cells treated for 4 hours with 1 µM BCM-7.

Genomic DNA was extracted from samples with the Easy DNA kit (Invitrogen K1800-01) using the appropriate protocol for cell lines. Fragmentation was performed on Covaris S2 with the following settings: duty cycle 10%, intensity 5, 200 cycles per burst during 200 sec. Fragments were obtained having an average length of 200 bp. The power mode is frequency sweeping, temperature 6-8 °C, water level 12. A maximum of 5 µg was loaded in 130 µl Tris- EDTA in a microtube with AFA intensifier. For samples with less DNA input (down to 500 ng) the DNA was diluted 1:5 in TrisEDTA. DNA with an input from 5-3 µg was analysed on the Agilent 2100 using a DNA 1000 chip. DNA with an input lower than 3 µg was concentrated in a rotary evaporator to 25 µl and the fragment distribution was checked on a high sensitivity DNA chip. Methylated DNA was captured using the MethylCap kit (Diagenode, Belgium). The yield was typically between 0.5 and 8 ng of total captured DNA. Fragments were subsequently sequenced using an Illumina Genome Analyzer II. The concentrations of fragmented and captured DNA were determined on a Fluostar Optima plate reader with the Quant-iT PicoGreen dsDNA Assay Kit (Invitrogen P7589) at 480/520nm.

To prepare the DNA library, a DNA Sample Prep Master Mix Set 1 (NEB E6040) was used in combination with a Multiplexing Sample Preparation Oligo Kit (96 samples, Illumina PE-400-1001). The entire fragmented DNA was utilised and followed the NEB protocols, using the multiplexing sequencing adapters provided in the Multiplexing Sample Preparation Oligo Kit. Size selection of the library was carried out on a 2% agarose gel (Low Range Ultra Agarose Biorad 161-3107). A 1Kb Plus ladder (Invitrogen 10787-018) was used and a gel was run at 120 V for 2 hrs. A fragment of 300 bps +/- 50bps was excised and eluted on a Qiagen Gel Extraction Kit column (Qiagen 28704) and eluted in 23 µl EB.

The Illumina library amplification index protocol was used with the following alterations: 22 µl DNA was used and performed 21 cycles run. The sample was purified on a Qiaquick PCR Purification column (Qiagen 28101) and eluted in 50 µl EB, 1:5 diluted, and concentrated in a rotary evaporator to 10 µl. 1 µl was applied to an Agilent 2100 HS DNA chip and the concentration was determined by smear analysis on the Agilent 2100. The samples were diluted to 10 nM. After denaturation with NaOH the samples were diluted to 16 pM. The Paired-End flow cell was prepared according to the Cluster Station User Guide. Sequencing was performed according to the HiSeq user guide (performing a Multiplexed PE Run), with 2 x 51 cycles for the paired end runs.

Whole genome DNA MBD-seq revealed differentially methylated transcripts (DMTs), as defined by false discovery rate (FDR) <0.1 and ANOVA followed by post-hoc student's t-test (p<0.05). Transcripts included both genes and noncoding RNAs that were differentially methylated/transcribed. The epigenetic changes as well as the transcription changes induced by BCM-7 in specific biological or functionally relevant pathways were evaluated using the Ingenuity Pathway Analysis (IPA) tool and pathways exhibiting the highest impact were identified. The results are shown in Table 4. The changes in the epigenetic status of genes responsible for the lactose metabolism and lactose synthesis are also reported to be altered under BCM7, as shown in Figure 8 and 9.

**Table 4: List of Differentially Methylated Transcripts under the influence of BCM-7**

| | **Functional Ontology / Gene Ontology** | | | | |
|---|---|---|---|---|---|
| | **Digestion** | **Lactose metabolism** | **Lactose Biosynthetic pathway** | **Gastric Acid secretion** | **Galactose metabolism** |
| **1** | AKR1C1 | LCT | B4GALT2 | PGC | GKN1 |
| **2** | AKR1C2 | | LGALS1 2 | SCTR | GALK2 |
| **3** | CCKBR | | B4GALT1 | OXTR | GALR2 |
| **4** | HTR3A | | | VIPR1 | GALT |
| **5** | MLNR | | | SST | GALR1 |
| **6** | SLC15A1 | | | PPARGC1A | CHST1 |
| **7** | CTRB2 | | | | NPY |
| **8** | CTRB1 | | | | PYY |
| **9** | MEP1B | | | | |
| **10** | SULT2A1 | | | | |
| **11** | CELA3A | | | | |
| **12** | AMY1C | | | | |
| **13** | CTSE | | | | |
| **14** | CCKAR | | | | |
| **15** | CAPN9 | | | | |

### Example 8: Effect of BCM-7 on lactase levels in small intestine

NOD mice (male and female) were commenced on a diet enriched in beta-casein A1 or A2 milk protein from weaning. These diets were made by Specialty Feeds Pty. Ltd. (Australia) to ensure adequate composition and nutrition. Cohorts of mice (n=10) from each gender and diet were euthanased at 10 weeks or 20 weeks. At the time of dissection tissue samples were collected and stored at -80 °C in RNAlater™. 40 NOD mice were followed in this study: 10 per group (male/female: A1/A2).

RNA from cell culture for the analysis of RNA transcription was isolated using the RNAqueous®-4PCR kit from Ambion (Austin, TX). The procedure was same as described by the manufacturer's protocol. Isolated RNA was treated with DNase to purify the RNA followed by RNA quantification using an ND-1000 NanoDrop spectrophotometer. cDNA was synthesised as described previously using the first-strand cDNA synthesis from Roche (Indianapolis, IN). RNA (1 mg), dNTP mix (1 mM), random hexamer primers (60 mM), with sufficient molecular biology grade H₂0, were added to achieve a final sample volume of 13 ml. Each sample was denatured at 65 °C for 5 minutes and then placed on ice. Transcriptor RT (20 units/ml) (Roche), Protector RNase inhibitor (40 U/ml) (Roche), 5 Transcriptor Reverse Transcriptase Reaction Buffer (Roche), and molecular biology grade H₂0, were added and the final volume was adjusted to 20 ml. This was followed by incubation in a PTC Thermocycler (MJ Research, St. Bruno, QC, Canada) at 25 °C for 10 minutes and 55°C for 30 minutes. Lastly, the reverse-transcriptase enzyme was inhibited by incubation at 85 °C for 5 minutes.

The qRT-PCR assay was performed on triplicate samples using a LightCycler 480 qRT-PCR machine from Roche (Trivedi et al., Mol. Pharmcol., 2014). qRT-PCR was performed using 5 ml of cDNA template, 10 mM sense and antisense primers, 10 ml SYBR Green I Master from Roche, as well as dH₂0 in a final volume of 20 ml. The primers used for this purpose were forward 5'-GGAGTGTCACCCACAGACAG-3' and reverse 5'-GAACACAAGCTACACGGGGA-3'. The samples were put through the following protocol: incubation for 5 minutes at 95 °C, and then 45 cycles of 95 °C for 10 seconds, 60 °C for 20 seconds, and 72 °C for 30 seconds, followed by a single cycle of 95 °C for 5 seconds, 1 minute at 65 °C, and 97 °C for the melting curve, followed by cooling at 40 °C for 90 seconds. No template controls (NTC) were run on the plate, and the dissociation curves were generated to determine the nonspecific products and this was normalised to avoid any non-specific amplification. Data were analyzed using the Roche quantification method D(DCt) and were normalised to beta-actin levels. The results are shown in Figure 10.

Although the invention has been described by way of example, it should be appreciated that variations and modifications may be made without departing from the scope of the invention as defined in the claims. Furthermore, where known equivalents exist to specific features, such equivalents are incorporated as if specifically referred in this specification.

The present disclosure includes the embodiments set out in the following numbered clauses:
1. The use of a composition for preventing or reducing the symptoms of lactose intolerance in an animal, where the composition contains beta-casein, and where the beta-casein comprises at least 75% by weight beta-casein A2.
2. The use according to clause 1, wherein the beta-casein comprises at least 90% by weight beta-casein A2.
3. The use according to clause 1 or clause 2, wherein the beta-casein comprises 100% beta-casein A2.
4. The use according to any one of clauses 1 to 3, wherein the composition is milk or a milk product.
5. The use according to clause 4, wherein the milk is fresh milk, milk powder, liquid milk reconstituted from powder, skim milk, homogenised milk, condensed milk, evaporated milk, pasteurised milk, or non-pasteurised milk.
6. The use according to clause 4, wherein the milk product is cream, yoghurt, quark, cheese, butter, or ice cream.
7. The use according to any one of clauses 1 to 6, wherein the symptoms of lactose intolerance include one or more of abdominal bloating and cramps, flatulence, diarrhoea, nausea, rumbling stomach, and vomiting.
8. The use according to any one of clauses 1 to 7, wherein the prevention or reduction of the symptoms of lactose intolerance is an acute response to consumption of the composition by the animal.
9. The use according to any one of clauses 1 to 7, wherein the composition predisposes the animal to preventing or reducing the symptoms of lactose intolerance on future exposure to lactose.
10. The use according to any one of clauses 1 to 9, wherein the animal is a human, dog, or cat.
11. A composition for preventing or reducing the symptoms of lactose intolerance in an animal which composition contains beta-casein and where the beta-casein comprises at least 75% by weight beta-casein A2.
12. The use of milk in the manufacture of a composition for preventing or reducing the symptoms of lactose intolerance in an animal where the milk contains beta-casein and where the beta-casein comprises at least 75% by weight beta-casein A2.
13. The use of beta-casein A2 in the manufacture of a composition for preventing or reducing the symptoms of lactose intolerance in an animal where the composition comprises at least 75% by weight beta-casein A2.
14. The use according to clause 13, wherein the beta-casein A2 is a component of milk.
15. A method of preventing or reducing the symptoms of lactose intolerance in an animal comprising the consumption by the animal of a composition containing beta-casein, or providing the composition to the animal for consumption, where the beta-casein comprises at least 75% by weight beta-casein A2.

## Claims

1. A composition for use in preventing or minimising epigenetic changes to one or more genes responsible for regulating the activity and levels of enzymes involved in digestive processes and gut function in an animal, wherein the composition contains beta-casein and wherein the beta-casein comprises at least 75% by weight of beta-casein variants having proline at position 67 of the beta-casein amino acid sequence .

2. A composition as claimed in claim 1, wherein preventing or minimising epigenetic changes to the one or more genes reduces the risk of developing symptoms of lactose intolerance.

3. A composition as claimed in claim 2, wherein the symptoms include one or more of abdominal bloating, abdominal cramps, flatulence, diarrhoea, nausea, rumbling stomach, and vomiting.

4. A composition as claimed in any one of claims 1 to 3, wherein the one or more genes are selected from the group comprising AKR1C1, AKR1C2, CCKBR, HTR3A, MLNR, SLC15A1, CTRB2, CTRB1, MEP1B, SULT2A1, CELA3A, AMY1C, CTSE, CCKAR, CAPN9, LCT, B4GALT2, LGALS12, B4GALT1, PGC, SCTR, OXTR, VIPR1, SST, PPARGC1A, GKN1, GALK2, GALR2, GALT, GALR1, CHST1, NPY, and PYY.

5. A composition as claimed in any one of claims 1 to 4, wherein the beta-casein comprises at least 90%, preferably at least 99%, by weight beta-casein A2.

6. A composition as claimed in any one of claims 1 to 5, wherein the beta-casein comprises less than 25%, preferably less than 10%, more preferably less than 1%, by weight beta-casein A1.

7. A composition as claimed in any one of claims 1 to 6, which is milk or a milk product.

8. A composition as claimed in claim 7, wherein the milk or milk product is obtained from bovine cows that are known to have the beta-casein A2A2 genotype.

9. A composition as claimed in claim 7 or 8, wherein the milk is fresh milk, milk powder, liquid milk reconstituted from powder, skim milk, homogenised milk, condensed milk, evaporated milk, pasteurised milk, or non-pasteurised milk.

10. A composition as claimed in claim 7 or 8, wherein the milk product is cream, yoghurt, quark, cheese, butter, infant formula, or ice cream.

11. A composition as claimed in any one of claims 1 to 10, wherein the animal is a human, a dog, or a cat.
